# EUROPEAN PATENT SPECIFICATION

(11) **EP 2 380 586 B1**
(45) Date of publication and mention of the grant of the patent: **15.07.2015**
(21) Application number: 10075581.8
(22) Date of filing: 05.10.2004
(51) Int. Cl.: A61K 45/06, A61K 38/55, A61K 38/34, A61K 31/198, A61L 31/10, A61L 31/14, A61L 31/16, A61P 41/00

(54) **R-Lys-X Oligopeptides as coating material for medical products**
R-Lys-X Oligopeptide als Beschichtungsmaterial für Medizinprodukte
Oligopeptides R-Lys-X servant de matériau d'enrobage pour des produits médicaux

(30) Priority: 07.10.2003 US 680035
(43) Date of publication of application: 26.10.2011
(62) Divisional of application: 04765826.5
(73) Proprietor: Hemoteq AG, 52146 Würselen (DE)
(72) Inventor: Hoffmann, Erika, 52249 Eschweiler (DE); Hoffmann, Michael, 52249 Eschweiler (DE); Horres, Roland, 52223 Stolberg (DE)
(74) Representative: Arth, Hans-Lothar

(56) References cited:
- WO-A1-03/034944
- WO-A1-03/061689
- WO-A2-00/56353
- WO-A2-01/58526
- WO-A2-02/083176
- WO-A2-03/020223
- WO-A2-03/044053
- BEOHAR NIRAT ET AL: "Inhibition of balloon injury mediated apoptosis with local delivery of a caspase inhibitor", JOURNAL OF THE AMERICAN COLLEGE OF CARDIOLOGY, vol. 37, no. 2 Supplement A, February 2001 (2001-02), page 44A, XP009043076, & 50TH ANNUAL SCIENTIFIC SESSION OF THE AMERICAN COLLEGE OF CARDIOLOGY; ORLANDO, FLORIDA, USA; MARCH 18-21, 2001 ISSN: 0735-1097
- KIM SUN CHEUN ET AL: "Caspase-3-dependent apoptosis in vascular smooth muscle cell by proteasome inhibition.", JOURNAL OF CARDIOVASCULAR PHARMACOLOGY, vol. 42, no. 4, October 2003 (2003-10), pages 554-560, XP009043075, ISSN: 0160-2446
- LEE DENNIS ET AL: "Potent and selective nonpeptide inhibitors of caspases 3 and 7", JOURNAL OF MEDICINAL CHEMISTRY, vol. 44, no. 12, 7 June 2001 (2001-06-07), pages 2015-2026, XP002315130, ISSN: 0022-2623
- ASHWELL S: "Caspases: Recent advances in small molecule inhibitors", EXPERT OPINION ON THERAPEUTIC PATENTS 2001 UNITED KINGDOM, vol. 11, no. 10, 2001, pages 1593-1603, XP002315131, ISSN: 1354-3776
- WIENEKE H ET AL: "Therapeutic potential of active stent coating", EXPERT OPINION ON INVESTIGATIONAL DRUGS 01 MAY 2003 UNITED KINGDOM, vol. 12, no. 5, 1 May 2003 (2003-05-01), pages 771-779, XP002315159, ISSN: 1354-3784
- BEOHAR NIRAT ET AL: "Antirestenotic effects of a locally delivered caspase inhibitor in a balloon injury model.", CIRCULATION. 6 JAN 2004, vol. 109, no. 1, 6 January 2004 (2004-01-06), pages 108-113, XP000002657509, ISSN: 1524-4539

## Description

### Field of the invention

The present invention relates to a pharmaceutical composition comprising a compound of the general formula R-Lys-X, methods for coating medical products using said compounds of general formula R-Lys-X and medical products coated with said compounds of general formula R-Lys-X.

### Background of the invention

In connection with coronary interventions and especially with the percutaneous transluminal coronary angioplasty (PTCA) it was demonstrated that this kind of non-surgical therapy is limited because of a restenosis rate of up to 35%. Several investigations show that the balloon angiography and also the stent implantation causes injuries and the tear of plaques and vascular walls, leading to neointimal hyperplasty and proliferation of smooth muscle cells.

Said smooth muscle cells generate an extracellular matrix in the newly formed intima. Furthermore, the injuries cause local inflammations and the migration of lymphocytes, macrophages and monocytes into the newly formed intima. This neointimal proliferation causes restenosis and methods are desired which reduce the risk of restenosis by controlling the proliferation and diminishing the inflammatory processes.

Object of the present invention is to provide compounds and pharmaceutical compositions for the reduction of restenosis, coating of medical products which reduce the risk of restenosis and methods for manufacturing said coated medical products.

The object is solved by the teaching of the independent claims. Further advantageous embodiments of the present invention are evident from the dependent claims, the description and the examples.

### Description of the invention

The present invention relates to the use of at least one compound of the general formula R-Lys-X wherein X represents an oligopeptide selected from the group comprising Pro-Thr, Pro-Ala, Pro-Arg, Pro-Asn, Pro-Asp, Pro-Cys, Pro-Glu, Pro-Gln, Pro-Gly, Pro-His, Pro-Ile, Pro-Leu, Pro-Lys, Pro-Met, Pro-Phe, Pro-Pro, Pro-Ser, Pro-Trp, Pro-Thr-Thr, Pro-Thr-Val, Pro-Thr-Ala, Pro-Thr-Arg, Pro-Thr-Asn, Pro-Thr-Asp, Pro-Thr-Cys, Pro-Thr-Glu, Pro-Thr-Gln, Pro-Thr-Gly, Pro-Thr-His, Pro-Thr-Ile, Pro-Thr-Leu, Pro-Thr-Lys, Pro-Thr-Met, Pro-Thr-Phe, Pro-Thr-Pro, Pro-Thr-Ser, and Pro-Thr-Trp and wherein R represents a peptide comprising the tetrapeptide His-Phe-Arg-Trp and/or wherein R represents a peptide comprising the tripeptide Phe-Arg-Trp and/or wherein R represents a peptide comprising the tripeptide His-Phe-Arg for the preparation of a pharmaceutical composition, the use of said at least one compound of said general formula R-Lys-X or said pharmaceutical composition for coating surfaces of medical products, especially of stents. Furthermore, the present invention relates to medical products coated according to the invention coating method, especially to stents coated according to the inventive methods.

Caspases are widely conserved proteases considered to be essential effectors of apoptosis.

A wide range of caspase inhibitors are known of which peptidic caspase inhibitors such as benzyloxycarbonyl-Val-Ala-Asp-fluoromethyl ketone or Ile-Glu-Thr-Asp-fluoromethyl ketone are the most popular examples.

Abbreviations used for protecting groups include: Z- (or z-), for I benzyloxycarbonyl; BOC (or boc), for t-butyloxycarbonyl; Bzl, for benzyl; Fmoc, for 9-fluorenyloxycarbonyl; Ac, for acetyl; FMK (or fmk), for fluoromethyl ketone; CMK (or cmk), for chloromethyl ketone.

The caspase inhibitors can be purchased from Enzyme Systems (Livermore CA).

The above mentioned caspase inhibitors can be used for the preparation of a pharmaceutical composition. Said pharmaceutical composition can furthermore be used for the coating of medical products such as artificial hearts, heart parts, lungs, arteries, veins, aortas, heart valves, corpse veins, valves, container, bags, cans, needles, catheter and parts especially artificial parts for the cardiovascular system and the extracorporeal circulation, surgical implants such as stents or catheters and devices for analytical purposes such as test tubes, titer plates, micro titer plates, well plates, analytical chips or material for chromatography such as gels, silica gels, columns, alumina, sepharose gels and the like. Most preferable are stents to be coated with a coating mixture such as the pharmaceutical composition mentioned above.

As protecting groups, benzyloxycarbonyl, fluoromethyl ketone, chloromethyl ketone and *t*-butoxycarbonyl are most preferred.

It is also preferred that at least one amino acid of the above-mentioned caspase inhibitors has D-configuration, especially if one amino acid of Tyr-Val-Ala-Asp has D-configuration.

Neuropeptides as the proopiomelanocortin peptides (POMC), especially alpha-, beta- and gamma- melanocyte-stimulating hormone (MSH), more especially alpha-MSH, and Adrenocorticotropin (ACTH) and their related tripeptides (KPV), are known to have anti-inflammatory and immunosuppressive effects on the endothelial cells (Broad medical research program for the Eli and Edythe L. Broad Foundation; Kucharzik 2003). These properties reside in the C-terminal part of the tridecapeptide alpha-MSH and KPVs, which consists of three amino acids Lys-Pro-Val (Catania and Lipton, Endocrin. Rev. 1993, 14, 564-578; Bhardvaj et al., J. Immunol. 1996, 156, 2517-2521). MSH is structurally related to ACTH and is biologically generated from the precursor POMC. The two different species of MSH, α-MSH and β-MSH, have the first 13 amino acids in common with ACTH. Plasmalipotropin (LPH) and Cardiotropinlike peptide (CLIP) originate also from the precursor POMC and are presumed to have positive effects (Clin. Endocrin. & Metabol. 2001, 86(7); 2997-3000).

Thus, another aspect of the present disclosure relates to the use of compounds derived from the family of POMC-peptides is alpha-, beta- or gamma-MSH, ACTH, LPH or CLIP or protected, acylated, acetylated derivatives of said compounds of formula as claimed in claim 1 for the coating of surfaces of medical product.

Some caspase inhibitors can be represented by the formula R-Lys-X wherein X represents an oligopeptide selected from the group comprising Pro-Thr, Pro-Ala, Pro-Arg, Pro-Asn, Pro-Asp, Pro-Cys, Pro-Glu, Pro-Gln, Pro-Gly, Pro-His, Pro-Ile, Pro-Leu, Pro-Lys, Pro-Met, Pro-Phe, Pro-Pro, Pro-Ser, Pro-Trp, Pro-Thr-Thr, Pro-Thr-Val, Pro-Thr-Ala, Pro-Thr-Arg, Pro-Thr-Asn, Pro-Thr-Asp, Pro-Thr-Cys, Pro-Thr-Glu, Pro-Thr-Gln, Pro-Thr-Gly, Pro-Thr-His, Pro-Thr-Ile, Pro-Thr-Leu, Pro-Thr-Lys, Pro-Thr-Met, Pro-Thr-Phe, Pro-Thr-Pro, Pro-Thr-Ser, and Pro-Thr-Trp and wherein R represents a peptide comprising the tetrapeptide His-Phe-Arg-Trp and/or wherein R represents a peptide comprising the tripeptide Phe-Arg-Trp and/or wherein R represents a peptide comprising the tripeptide His-Phe-Arg. It was surprisingly found that not only caspase inhibitors but also oligopeptides and peptides of said general formula R-Lys-X are able to solve the problem underlying the invention. Said compounds including the caspase inhibitors which can also be used for the preparation of the pharmaceutical composition and for coating the surface of medical products are represented by the general formula R-Lys-X, wherein X represents an oligopeptide selected from the group comprising Pro-Thr, Pro-Ala, Pro-Arg, Pro-Asn, Pro-Asp, Pro-Cys, Pro-Glu, Pro-Gln, Pro-Gly, Pro-His, Pro-Ile, Pro-Leu, Pro-Lys, Pro-Met, Pro-Phe, Pro-Pro, Pro-Ser, Pro-Trp, Pro-Thr-Thr, Pro-Thr-Val, Pro-Thr-Ala, Pro-Thr-Arg, Pro-Thr-Asn, Pro-Thr-Asp, Pro-Thr-Cys, Pro-Thr-Glu, Pro-Thr-Gln, Pro-Thr-Gly, Pro-Thr-His, Pro-Thr-Ile, Pro-Thr-Leu, Pro-Thr-Lys, Pro-Thr-Met, Pro-Thr-Phe, Pro-Thr-Pro, Pro-Thr-Ser, and Pro-Thr-Trp and wherein R represents a peptide comprising the tetrapeptide His-Phe-Arg-Trp and/or wherein R represents a peptide comprising the tripeptide Phe-Arg-Trp and/or wherein R represents a peptide comprising the tripeptide His-Phe-Arg. The compounds of general formula R-Lys-X can be regarded as caspase inhibitors, too. Thus, as used herein, the term caspase inhibitors shall refer to the caspase inhibitors known from literature and also to the compounds of formula R-Lys-X.

Preferably, R represents a peptide having 3 - 50 amino acids, more preferably R represents a peptide having 5 - 35 amino acids, still more preferably R represents a peptide having 6 - 20 amino acids, further still more preferably R represents a peptide having 7 - 15 amino acids, still more preferably R represents a peptide having 8 - 12 amino acids, and most preferably R represents a peptide having 9 - 11 amino acids. Also most preferably R is a peptide of 10 amino acids.

Furthermore, R represents a peptide comprising the tetrapeptide His-Phe-Arg-Trp or the tripeptides Phe-Arg-Trp or His-Phe-Arg.

Further preferred embodiments comprise compounds of general formula R-Lys-X wherein at least one amino acids of the residues R and/or X has D-configuration. More preferred are compounds wherein X comprises of L amino acids and R contains at least one D amino acid. Another more preferred embodiment of the present invention comprises compounds of general formula R-Lys-X wherein all amino acids of the residue X have L-configuration and all amino acids of the residue R have D-configuration. Within all mentioned embodiments it is also advantageous that the amino acid -Lys- in R-Lys-X has L-configuration.

According to the nomenclature of peptides, R is the residue leading to the N-terminal end of the peptide and X is the residue bound to the C-terminal end of the amino acid -Lys- in R-Lys-X.

Both ends, the C-terminal and the N-terminal end of the compound of general formula R-Lys-X, may be protected with common amino or carboxyl protecting groups such as acyl groups. Preferred amino protecting groups are acyl groups, such as formyl, acetyl, propionyl and preferably the acetyl group. Preferred protecting groups for carboxylic acids are monoalkylamino groups, dialkylamino groups, alkoxy groups, fluoromethyl ketones and chloromethyl ketones. Said protecting groups can be present at the C-terminal or N-terminal end or at both ends or at none of them.

The present invention comprises compounds of I general formula R-Lys-X wherein X represents an oligopeptide selected from the group comprising Pro-Thr, Pro-Val, Pro-Ala, Pro-Arg, Pro-Asn, Pro-Asp, Pro-Cys, Pro-Glu, Pro-Gln, Pro-Gly, Pro-His, Pro-Ile, Pro-Leu, Pro-Lys, Pro-Met, Pro-Phe, Pro-Pro, Pro-Ser, Pro-Trp, Pro-Thr-Thr, Pro-Thr-Val, Pro-Thr-Ala, Pro-Thr-Arg, Pro-Thr-Asn, Pro-Thr-Asp, Pro-Thr-Cys, Pro-Thr-Glu, Pro-Thr-Gln, Pro-Thr-Gly, Pro-Thr-His, Pro-Thr-Ile, Pro-Thr-Leu, Pro-Thr-Lys, Pro-Thr-Met, Pro-Thr-Phe, Pro-Thr-Pro, Pro-Thr-Ser, and Pro-Thr-Trp. The afore-mentioned dipeptides and tripeptides and Pro may also be protected with one of the above-mentioned protecting groups for carboxylic acids, especially fluoromethyl ketones and chloromethyl ketones.

Furthermore, the following compounds are preferred:
R"-His-Phe-Arg-Trp-R'-Lys-X,
R"-His-Phe-Arg-Trp-R'-Lys-Pro-Thr-X',
R"-Phe-Arg-Trp-R'-Lys-X,
R"-Phe-Arg-Trp-R'-Lys-Pro-Thr-X',
R"-His-Phe-Arg-R'-Lys-X,
and R"-His-Phe-Arg-R'-Lys-Pro-Thr-X',
wherein X' represents a hydroxyl group, an amino group, a monoalkyl or dialkylamino group, an alkoxy group, and wherein R' represents an oligopeptide of 1 - 10 amino acids and R" is selected from the group comprising hydrogen, acyl group, acetyl group, an amino acid or a peptide with 1 - 60 amino acids.

Most preferably, X' represents one C-terminal protected or unprotected amino acid group. Furthermore, the L-configuration of X' is preferred.

R' is more preferable selected from the group comprising oligopeptide sequences of 1 - 5, still more preferably of 1 - 3 and most preferably of one or two amino acid residues. Furthermore, the L-configuration of the amino acids of R' is preferred.

In addition thereto, N-terminal protected or unprotected peptides consisting of 1 - 40 amino acids, preferably 2 - 30, more preferably 3 - 20, still more preferably 3 - 13, still more preferably 4 - 7, and most preferably 5 or 6 are useful as residue R". Furthermore, it is advantageous if at least one amino acid of the residue R" has D-configuration. It is more advantageous if 10% and still more advantageous if 50% and most advantageous if more than 90% of the amino acids of R" have D-configuration.

Compounds of the formula Lys-X, wherein X represents a hydroxyl group, an amino group, an alkoxy group, Proline or Pro-Thr are known to have anti-inflammatory properties (WO 02/064131) and are suitable for coating compositions on medical devices. Derivatisations at the side chains of Lysine or Threonine are also possible without loosing the therapeutic character, chain extention up to the length of alpha-MSH and more offers a broad variety of derivatives.

Another aspect of the present invention relates to methods for coating medical products. Such methods comprise the steps of:
a) providing a surface of a medical product,
b) coating said surface with a coating composition comprising at least one compound of formula R-Lys-X wherein represents an oligopeptide selected from the group comprising Pro-Thr, Pro-Ala, Pro-Arg, Pro-Asn, Pro-Asp, Pro-Cys, Pro-Glu, Pro-Gln, Pro-Gly, Pro-His, Pro-Ile, Pro-Leu, Pro-Lys, Pro-Met, Pro-Phe, Pro-Pro, Pro-Ser, Pro-Trp, Pro-Thr-Thr, Pro-Thr-Val, Pro-Thr-Ala, Pro-Thr-Arg, Pro-Thr-Asn, Pro-Thr-Asp, Pro-Thr-Cys, Pro-Thr-Glu, Pro-Thr-Gln, Pro-Thr-Gly, Pro-Thr-His, Pro-Thr-Ile, Pro-Thr-Leu, Pro-Thr-Lys, Pro-Thr-Met, Pro-Thr-Phe, Pro-Thr-Pro, Pro-Thr-Ser, and Pro-Thr-Trp and wherein R represents a peptide comprising the tetrapeptide His-Phe-Arg-Trp and/or wherein R represents a peptide comprising the tripeptide Phe-Arg-Trp and/or wherein R represents a peptide comprising the tripeptide His-Phe-Arg.

The coating layer comprising the caspase inhibitor can be applied directly on the surface, normally an uncoated surface of the medical product. It is also possible to generate a first coating layer comprising of biologically stable and/or biodegradable polymers and to coat said first layer with a second layer comprising at least one compound of the general formula R-Lys-X, wherein R and X have the meanings as defined above. Said first coating layer may further comprise at least one anti-inflammatory, anti-prolific, anti-thrombotic, and/or anti-coagulative agent or said first coating layer may completely or mainly consist of said anti-inflammatory, anti-prolific, anti-thrombotic, and/or anti-coagulative agent. Preferably, the anti-inflammatory, anti-prolific, anti-thrombotic, and/or anti-coagulative agents as listed below are used with the coating methods.

Furthermore, it is advantageous to provide another layer as the outermost layer over or on top of the layer comprising at least one compound of general formula R-Lys-X, wherein R and X have the meanings as defined above. The layer or the layers comprising a biologically stable polymer, a biodegradable polymer, at least one compound of said general formula R-Lys-X may further comprise at least one anti-inflammatory, anti-prolific, anti-thrombotic, and/or anti-coagulative agent.

Preferably are coatings consisting of one or two layers. The layers, preferably the outermost layer can be designed in a way capable of allowing controlled release of the at least one anti-inflammatory, anti-prolific, anti-thrombotic, and/or anti-coagulative agent and/or the at least one compound of general formula R-Lys-X wherein R and X have the meanings as defined above.

It is also advantageous that the layer below or on top of the layer comprising the at least one compound of general formula R-Lys-X further comprises at least one anti-inflammatory, anti-prolific, anti-thrombotic, and/or anti-coagulative agent or that said layer completely or mainly consists of said anti-inflammatory, anti-prolific, anti-thrombotic, and/or anti-coagulative agent. Thus two embodiments are preferred: a) first layer consisting mainly or completely of at least one anti-inflammatory, anti-prolific, anti-thrombotic, and/or anti-coagulative agent, preferably taxol® (paclitaxel), or a first layer consisting mainly of a biostable and/or biodegradable polymer, preferably selected from the group mentioned below, said layer comprising at least one anti-inflammatory, anti-prolific, anti-thrombotic, and/or anti-coagulative agent, preferably taxol® (paclitaxel), and a second layer formed on said first layer containing said compound of general formula R-Lys-X or b) embodiments wherein the first and second layer is exchanged with each other Thus, it is possible to have one layer consisting of or mainly comprising said at least one anti-inflammatory, anti-prolific, anti-thrombotic, and/or anti-coagulative agent. It is also possible to have that at least one anti-inflammatory, anti-prolific, anti-thrombotic, and/or anti-coagulative agent incorporated into at least one layer comprising the biostabile and/or biodegradable polymer and/or the at least one compound of general formula R-Lys-X, wherein R and X have the meanings as defined above. Furthermore, it is possible to have different anti-inflammatory, anti-prolific, anti-thrombotic, and/or anti-coagulative agents in different layers or to have the same anti-inflammatory, anti-prolific, anti-thrombotic, and/or anti-coagulative agent in different layers. Said compounds of general formula R-Lys-X can be released from different layers with different releasing rates or from the same layer with different releasing rates. The releasing rates are adjusted and controlled by the properties of the used polymer(s).

Another preferred embodiment comprises a layer only consisting of at least one anti-inflammatory, anti-prolific, anti-thrombotic, and/or anti-coagulative agent and at least one compound of general formula R-Lys-X wherein R and X have the meanings as defined above. Said embodiments preferably have one or two layers. The embodiments with two layers have one biostable and/or biodegradable polymer layer below or on top of said layer consisting only of at least one anti-inflammatory, anti-prolific, anti-thrombotic, and/or anti-coagulative agent and at least one compound of general formula R-Lys-X.

The term "biostable and biodegradable polymer" means either a composition of at least one biostable polymer and at least one biodegradable polymer or at least one block-polymer consisting of sequences which are biostabile and of sequences which are biodegradable.

The term "mainly" has the meaning of at least 85%, preferably at least 90%, more preferably more than 95%, still more preferably at least 98%, and most preferably more than 99%.

The layer containing said caspase inhibitor and/or said compound of general formula R-Lys-X and/or said anti-inflammatory, anti-prolific, anti-thrombotic, and/or anti-coagulative agent can be formed directly on the normally not hemocompatible surface of the medical product, or on a first layer applied on the surface of the medical product. On top of the layer containing said compound of general formula R-Lys-X and/or said anti-inflammatory, anti-prolific, anti-thrombotic, and/or anti-coagulative agent another layer can be generated.

Said outermost layer preferably comprises biologically stable and/or biodegradable polymers and more preferably consists mainly of biologically stable and/or biodegradable polymers. Moreover, said outermost layer may contain another anti-inflammatory, anti-prolific, anti-thrombotic, and/or anti-coagulative agent which may be identical or different from the agent used in a layer under said outermost layer. Another preferred embodiment contains an anti-inflammatory, anti-prolific, anti-thrombotic, and/or anti-coagulative agent only in the outermost layer.

The surface of the medical product may consist of metals, such as stainless steel or titan, chromium, tungsten, vanadium, gold, copper, nitinol, molybdenum, alloys, ceramics, minerals, silicate materials such as glass, natural materials such as tissue, cells, biopolymers, synthetic polymers or plastics such as Teflon® (tetrafluoroethylene), PCV (polyvinyl chloride), polyethylene terephthalates, polyethylene, polypropylene, polyamides, polyurethanes, polycarbonates, polysulfones, polyarylsulfones, polyethersulfones, modified polysulfones, modified polyarylsulfones, modified polyethersulfones, hydrophilised polysulfones hydrophobised polysulfones, polyether etherketones, silicones, polystyrene, polymethyl methacrylates, polyvinylidene fluorides and mixtures or copolymers of the aforementioned plastics and synthetic polymers.

As biostabile polymers may be used polyacrylic acid, polyacrylates, polymethylmethacrylates, polybutylmethacrylates, polyacrylamides, polyacrylonitriles, polyamides, polyether amides, polyethylene amines, polyimides, polycarbonates, polycarbourethanes, polyvinylketones, polyvinyl halides, polyvinyliden halides, polyvinyl ether, aromatic polyvinyls, polyvinyl esters, polyvinyl pyrollidones, polyoxymethylene, polyethylene, polypropylene, polytetrafluoroethylene, polyurethanes, polyolefin-elastomers, polyisobutylene, EPDM-gum, fluorosilicones, carboxymethyl chitosan, polyethylene terephtalat, polyvalerate, carboxymethyl celluloses, cellulose, rayon, rayontriacetate, cellulosenitrates, cellulose acetates, hydroxyethyl celluloses, cellulose butyrates, cellulose acetat-butyrates, ethylvinyl acetat-copolymeres, polysulfones and their modified homologues, epoxy resins, ABS resins, EPDM-gum, silicones such as polysiloxanes, polyvinyl halides, and copolymeres, cellulose ether, cellulose triacetate, chitosan and copolymers and/or mixtures of the aforementioned polymers.

The biodegradable polymers can be selected from the group comprising polyvalerolactone, poly-ε-decalactone, polylactides, polyglycolides, copolymers of polylactide and polyglycolide, poly-ε-caprolacton, polyhydroxy butyric acid, polyhydroxy butyrate, polyhydroxy valerate, polyhydroxy butyrate-co-valerate, poly(1,4-dioxan-2,3-dione), poly(1,3-dioxan-2-one), poly-para-dioxanone, polyanhydrides such as polymaleic acid anhydride, polyhydroxy methacrylate, fibrin, polycyano acrylate, polycaprolacton dimethylacrylate, poly-β-maleic acid, polycaprolacton butyl-acrylate, multiblock polymers made of oligocaprolacton diole and oligodioxanon diole, polyether ester-multiblock polymers made of PEG and poly(butylenterephtalate, polypivotolactone, polyglycolic acid trimethyl-carbonate polycaprolacton-glycolide, poly(γ-ethylglutamate), poly(DTH-iminocarbonates), poly(DTE-co-DT-carbonates), poly(bisphenol A-iminocarbonates), polyorthoesters, polyglycolic acid trimethylcarbonates, polytrimethylcarbonates, polyiminocarbonates, poly(N-vinyl)-pyrrolidone, polyvinyl alcohols, polyester amides, glycolic polyester, polyphosphoesters, polyphosphazenes, poly[p-carboxyphenoxy)propane] polyhydroxypentanoic acid, polyanhydrides, polyethylenoxid-propylenoxid, smooth polyurethanes, polyurethanes bearing amino acid residues, polyether esters such as polyethylene oxid, polyalkenoxalates, polyorthoesters and copolymers thereof, carrageenanes, fibrinogen, starch, collagens, protein-based polymers, polyamino acids, synthetic polyamino acids, zein, modified zein, polyhydroxy alkanoates, pectinin acid, actinic acid, modified and unmodified fibrin and casein, carboxymethylsulfates, albumine, hyaluronic acid, heparan sulfates, heparin, chondroitin sulfates, dextranes, β-cydodextrines, copolymere with PEG and/or polypropylen glycol, gum arabicum, guar, gelatine, collagens, collagen-N-hydroxysuccinimid, derivatives, modifications, copolymers and/or mixtures of the aforementioned biodegradable polymers.

The anti-inflammatory, anti-prolific, anti-thrombotic, and/or anti-coagulative agent may be selected from the group comprising:
Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-hydroxy oxycyclophosphamide, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A and B, Mitoxanthrone, Azathioprin, Mycophenolatmofetil, c-myc-Antisense, b-myc-Antisense, Betulinsäure, Camptothecin, PI-88 (sulfated oligosaccharide), Melanocyte-stimulating hormone (α-MSH), activated protein C, IL1-ß-inhibitor, Thymosin α-1, fumaric acids and esters thereof, Calcipotriol, Tacalcitol, Lapachol, β-Lapachon, Podophyllotoxin, Betulin, podophyllic acids 2-ethylhydrazide, Molgramostim (rhuGM-CSF), Peginterferon α-2b, Lanograstim (r-HuG-CSF), Filgrastim, Macrogol, Dacarbazin, Basiliximab, Daclizumab, Selectin (Cytokin antagonist), CETP-Inhibitor, Cadherine, Cytokininhibitoren, COX-2-inhibitor, NFkB, Angiopeptin, Ciprofloxacin, Camptothecin, Fluroblastin, monoclonal antibodies which inhibit proliferation of muscle cells, bFGF-antagonists, Probucol, Prostaglandine, 1,11-dimethoxycanthin-6-one, 1-hydroxy-11-methoxycanthin-6-one, Scopolectin, Colchicin, NO donors such as pentaerythrityltetranitrate and Syndnoeimine, S-nitroso derivatives, Tamoxifen, Staurosporin, β-Estradiol, α-Estradiol, Estriol, Estron, Ethinylestradiol, Fosfestrol, Medroxyprogesteron, Estradiolcypionate, Estradiolbenzoate, Tranilast, Kamebakaurin and other terpenoides which are used in cancer therapy, Verapamil, Tyrosin-Kinase-inhibitors (Tyrphostine), Cyclosporin A, Paclitaxel and derivatives thereof such as 6-α-hydroxy-Paclitaxel, Baccatin, Taxotere, synthetic macrocyclic oligomers of carbonsuboxids (MCS) and derivatives thereof, Mofebutazon, Acemetacin, Diclofenac, Lonazolac, Dapson, o-Carbamoylphenoxy acetic acid, Lidocain, Ketoprofen, Mefenaminsäure, Piroxicam, Meloxicam, chloroquinphosphate, Penicillamin, Tumstatin, Avastin, D-24851, SC-58125, hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 protein, Bacitracin, Vitronectin-receptor antagonists, Azelastin, Guanidylcyclase-stimulator, inhibitors of metallproteinase-1 and 2, free nucleic acids, nucleic acids incorporated into virus hosts, DNA- and RNA-fragments, Plaminogen-activator inhibitor-1, Plasminogen-activator inhibitor-2, Antisense oligonucleotides, VEGF-inhibitors, IGF-1, antibiotics such as Cefadroxil, Cefazolin, Cefaclor, Cefotixin, Tobramycin, Gentamycin, Penicillines such as Dicloxacillin, Oxacillin, Sulfonamide, Metronidazol, antithrombotics such as Argatroban, Aspirin, Abciximab, synthetic Antithrombin, Bivalirudin, Coumadin, Enoxoparin, desulfated and N-reacetylated heparin, Tissue-Plasminogen-activator, Gpllb/llla-platelet membrane receptor, factor Xₐ-inhibitor antibody, Heparin, Hirudin, r-Hirudin, PPACK, Protamin, sodium salt of 2-methylthiazolidin-2,4-dicarboxylic acid (Thialin-Na), Prourokinase, Streptokinase, Warfarin, Urokinase, Vasodilatoren such as Dipyramidol, Trapidil, Nitroprusside, PDGF-antagonists such as Triazolopyrimidin and Seramin, ACE-inhibitors such as Captopril, Cilazapril, Lisinopril, Enalapril, Losartan, Thioproteaseinhibitoren, Prostacyclin, Vapiprost, Interferon α, ß and y, Histamin antagonists, Serotoninblocker, apoptosis inhibitors, apoptosis regulators such as p65, NF-kB or Bcl-xL-antisense-oligonucleotids, Halofuginon, Nifedipin, Tocopherol, Vitamin B1, B2, B6 and B12, folic acid, Tranirast, Molsidomin, Teepolyphenole, Epicatechingallat, Epigallocatechingallat, Boswellinic acid and its derivatives, Leflunomid, Anakinra, Etanercept, Sulfasalazin, Etoposid, Dicloxacyllin, Tetracyclin, Triamcinolon, Mutamycin, Procainimid, D24851, SC-58125, retinoic acid, Quinidin, Disopyrimid, Flecainid, Propafenon, Sotolol, Amidoron, natural and synthetic steroides such as Bryophyllin A, Inotodiol, Maquirosid A, Ghalakinosid, Mansonin, Streblosid, Hydrocortison, Betamethason, Dexamethason, none-steroidal substances (NSAIDS) such as Fenoporfen, lbuprofen, Indomethacin, Naproxen, Phenylbutazon and other antiviral agents such as Acyclovir, Ganciclovir and Zidovudin, antimycotics such Clotrimazol, Flucytosin, Griseofulvin, Ketoconazol, Miconazol, Nystatin, Terbinafin, antiprozoal agents such as Chloroquin, Mefloquin, Quinin, natural Terpenoides such as Hippocaesculin, Barringtogenol-C21-angelat, 14-Dehydroagrostistachin, Agroskerin, Agroskerin, Agrostistachin, 17-Hydroxyagrostistachin, Ovatodiolide, 4,7-oxycycloanisomelic acid, Baccharinoide B1, B2, B3 and B7, Tubeimosid, Bruceanole A, B and C, Bruceantinoside C, Yadanzioside N and P, Isodeoxyelephantopin, Tomenphantopin A and B, Coronarin A, B, C and D, Ursolic acid, Hyptatic acid A, Zeorin, Iso-Iridogermanal, Maytenfoliol, Effusantin A, Excisanin A and B, Longikaurin B, Sculponeatin C, Kamebaunin, Leukamenin A and B, 13,18-dehydro-6-alpha-Senecioyloxychaparrin, Taxamairin A and B, Regenilol, Triptolid, Cymarin, Apocymarin, Aristolochic acid, Anopterin, Hydroxyanopterin, Anemonin, Protoanemonin, Berberin, Cheliburinchloride, Cictoxin, Sinococulin, Bombrestatin A and B, Cudraisoflavon A, Curcumin, Dihydronitidin, Nitidinchloride, 12-beta-hydroxypregnadien 3,20-dione, Bilobol, Ginkgol, Ginkgolsäure, Helenalin, Indicin, Indicin-N-oxide, Lasiocarpin, Inotodiol, glycoside 1 a, Podophyllotoxin, Justicidin A and B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinoside, Ursolic acid, deoxypsorospermin, Psycorubin, Ricin A, Sanguinarin, Manwuweioic acid, methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A and B and sulfur containing amino acids such as cystin and salts thereof and/or mixtures of the above mentioned agents.

Another aspect of the present invention relates to medical products obtainable according to one of the coating methods described above. Most preferably, the coated medical products are stents.

Preferred anti-inflammatory, anti-prolific, anti-thrombotic, and/or anti-coagulative agents are: Tacrolimus, Pimecrolimus, PI 88, Paclitaxel and derivatives thereof, Trapidil, α- and β-Estradiol, 2-methylthiazolidin-2,4-dicarboxylic acid and salts thereof, preferably sodium salts, macrocyclic carbon suboxyd (MCS) and derivatives thereof, Sirolimus, fumaric acid and esters thereof, activated protein C, interleucin 1ß-inhibitors and melanocyte-stimulating hormone (α-MSH), cystin, Ellipticin, Bohemin, Indanocin, Colcemid and derivatives thereof, methionin and salts thereof and/or mixtures of the aforementioned agents. Taxol® (paclitaxel) is the most preferred anti-inflammatory, anti-prolific, anti-thrombotic, and/or anti-coagulative agent.

### Examples

The way of action of stents coated according to the present invention was investigated using animal models.

An increased amount of apoptotic smooth muscle cells in coronary arteries of pigs could be detected 30 minutes after a balloon angioplasty. Thereafter, the adventitia and the neointima were separately analyzed and different time-depending changes in the rate of apoptosis were measured. The highest levels of apoptotic smooth muscle cells, inflammatory cells, and fibroblast cells of the adventitia respectively were detected 18 hours, 6 hours and 7 days after PTCA (percutaneous transluminal coronary angioplasty). A quantitative determination of the rate of apoptosis in the different cell types and vessel wall layers after balloon angioplasty and stent implantation was conducted as follows:
Domestic pigs having a weight between 20 and 30 kg were fed with normal feed without the addition of fat supplementaries during the whole test. The pigs were kept fasting over night and were thereafter sedated using 30 mg/kg body weight of ketamine, 12 mg azepromazin and 1 ampoule of rubinol. 5 mg/kg of thiopental were administered before intubation. The pigs were given an artificial respiration by use of a mixture of 20% pure oxygen and 80% normal air after endotracheal intubation. Endotracheal intubation is a procedure by which a tube is inserted through the mouth down into the trachea (the large airway from the mouth to the lungs). After administration of 0.1 mg fentanyl and 2.5 mg aceproazin into the bolus, the anaesthesia was maintained by administration of 0.08 mg/kg fentanyl (0.05 mg/ml infusion). Procain-Penicillin G (200,000 lU/ml) and dihydro stretomycinsulfate (200 mg/10 kg body weight) were administered intramuscularly for the purpose of antibiotic protection.

Thereafter, an arteriotomy of the A. carotis communis dextra was carried out under sterile conditions and a 7F-channel was introduced. Puls, arterial blood pressure, and body temperature were measured during the whole operation. Additionally, the blood gases and the acid-base-metabolism were controlled in samples of arterial blood. After the administration of 200 IU/kg body weight of heparin and 250 mg/kg acetylsalicylic acid, a 7F catheter was inserted into the aorta ascendens. Additional 400 IU of heparin per hour were administered via infusion. The angiography of the right and left coronary artery was carried out by the use of non-ionic contrast agents after intracoronary administration of 200 µg nitroglycerin.

One artery of the left vascular system (either A. interventricularis or A. circumflexa) was randomly selected for stent implantation and the other artery was used for balloon angioplasty. The arteria coronaria was used as untreated control vessel.

A balloon having at least a balloon-vessel-ratio of 1.3 : 1 was used for balloon angioplasty in order to hurt the artery by overexpansion. The vessel was dilated (expanded) three times at the same position for 30 seconds and a pressure of 6 atm (atmospheres).

Thereafter, stents having a length of 15 mm were implanted according to standard methods. The diameter of the stent was selected in the way that a stent-vessel-ratio of 1.1 : 1 was obtained. During implantation, the stent balloon was blown up three times for 30 seconds applying a pressure of 6 bar.

An angiography of the right coronary artery was performed after a control angiography of the treated vessels had been carried out. Then, the catheter and the channel were removed and after ligation of the place of arteriotomy, the fascia and the skin were sewed up. Thereafter, the anaesthesia was stopped and the antibiotics trimethoprim and sulfadoxin together with the analgesic drug metamizol were administered. In addition thereto, 250 mg acetylsalicylic acid was given per day and per os (oral) after the intervention during the remaining live time of the animals in order to prevent acute or subacute thrombosis caused by the stent.

After 4 weeks a control angiography of the right and left coronary system was performed and an intravascular ultrasonic examination of the stent and the place treated with the dilated balloon was conducted.

Thereafter the pigs were euthanized by intravenous injection of 10 ml of a saturated potassium chloride solution. The hearts of the pigs were retained and washed with a sodium chloride solution. Thereafter, a pressure fixation was performed by use of buffered formaldehyde (4%) and about 100 - 110 mmHG perfusion pressure. Then, the coronary arteries of the heart were cut off, stored for 24 hours in buffered formaldehyde (2%) and thereafter in paraffin. The stent was removed using a microscope before storing the vessel in paraffin in order to prevent injury of the vessel.

In three test series the caspase inhibitors Tyr-Val-Ala-Asp (YVAD) or SYSMEHFRWGKPV or Ac-His-Phe-Arg-Lys-Pro-Asp-CMK were each locally administered during the test period via a perfusion balloon by means of a poly-lumen catheter. Said catheter consists of an infusion connector, a catheter body and distal infusion regions comprising 4 separate lumens.

One group of test animals received the caspase inhibitor (YVAD or SYSMEHFRWGKPV or Ac-His-Phe-Arg-Lys-Pro-Asp-CMK) while another group were kept untreated as control group. The neointimal proliferation was macroscopically assessed via IVUS whereby the analyses were carried out 4 weeks after balloon angioplasty and stent implantation. All IVUS measurements were evaluated off-line by means of a computer-based IVUS analysis system. The qualitative IVUS analysis comprises an assessment of the plaque composition (hard or smooth, thrombus, tear plaque or calcification respectively) and the eccentricity. The neointimal proliferation was calculated as average of 3 values. Moreover, histological investigations were performed. For this purpose, cuts of each segment of the artery were colored with hematoxylin-eosin and Verhoeff-van-Gieson in order to indicate injuries of the vessel caused by the intervention.

Quantitative evaluation of the injuries of the vessel and the neointimal response to the stent implantation was performed using the cuts colored according to Verhoeff-van-Gieson by applying a method created by Schwartz et al. Each wire of the stent was assessed and classified according to the severity of the injuries caused by this wire and the position of said wire in the histological layers of the vascular wall.

For the identification of macrophages and smooth muscle cells, anti-rabbit-macrophages-antibodies of mice (RAM 11, DAKO Corp.) and anti-rabbit-smooth-muscle-cells-alpha-actin-monoclonal-antibodies of mice were used. Proliferating cells were detected by marking the cuts with mouse antibodies against PCNA (proliferating cell nuclear antigen; clone PC 10, DAKO). For this purpose, the tissue was incubated for 1 hour together with primary antibodies at 37°C in a humidified chamber. The binding of the antibodies was achieved applying an indirect biotin-streptavadin horseradish peroxidase (Amersham) or alkaline phosphatase (Sigma) method. The methods were carried out according to the instructions of the supplier.

Finally, an in situ evaluation of apoptotic cells was performed. For this purpose the terminal transferase-mediated dUTP nick end labeling kit (TUNEL), a kit for displaying apoptosis in situ, was used according to the supplier's instructions. Thereby, positive controls were treated with DNAse after fixation and permeabilisation in order to cleave DNA and to obtain DNA strand pieces. Simultaneously, negative controls were stained with a staining solution (without terminal transferase) instead of the TUNEL reaction mixture. The binding of antibodies were visualized with diaminobenzidine (Pierce). The reaction with diaminobenzidine causes a brown color.

It could be demonstrated that said parts of the blood vessel which were treated with an apoptosis inhibitor (in the present case YVAD or SYSMEHFRWGKPV or Ac-His-Phe-Arg-Lys-Pro-Asp-CMK) showed a reduction of plaque volume to approximately 1/6 to 1/7, a reduction of maximum plaque area to approximately 1/3 and a reduction of the stenotisized (the area which comes into contact with the introduced stent) area to approximately 30% - 40% in comparison with the values obtained from the negative control group. 7 pigs were used for each group, the positive and the negative control group.

From the foregoing description, additional embodiments of the present invention will be immediately apparent to those skilled in the art.

## Claims

1. A medical product coated with a pharmaceutical composition comprising a compound of the formula R-Lys-X and at least one pharmaceutically acceptable carrier, polymer matrix, solvent and/or diluents, wherein X represents an oligopeptide selected from the group comprising Pro-Thr, Pro-Ala, Pro-Arg, Pro-Asn, Pro-Asp, Pro-Cys, Pro-Glu, Pro-Gln, Pro-Gly, Pro-His, Pro-Ile, Pro-Leu, Pro-Lys, Pro-Met, Pro-Phe, Pro-Pro, Pro-Ser, Pro-Trp, Pro-Thr-Thr, Pro-Thr-Val, Pro-Thr-Ala, Pro-Thr-Arg, Pro-Thr-Asn, Pro-Thr-Asp, Pro-Thr-Cys, Pro-Thr-Glu, Pro-Thr-Gln, Pro-Thr-Gly, Pro-Thr-His, Pro-Thr-Ile, Pro-Thr-Leu, Pro-Thr-Lys, Pro-Thr-Met, Pro-Thr-Phe, Pro-Thr-Pro, Pro-Thr-Ser, and Pro-Thr-Trp and wherein R represents a peptide comprising the tetrapeptide His-Phe-Arg-Trp and/or wherein R represents a peptide comprising the tripeptide Phe-Arg-Trp and/or wherein R represents a peptide comprising the tripeptide His-Phe-Arg

2. The medical product according to claim 1, wherein R represents a peptide comprising at least one amino acid having D configuration and/or wherein R represents a peptide consisting of amino acids having D configuration and/or R represents a peptide bearing an acyl group or acetyl group at the N-terminal end.

3. The medical product according to claim 1, wherein X represents the oligopeptide Pro-Thr.

4. The medical product according to claim 1, or 3, wherein X represents an oligopeptide bearing an amino group, a monoalkyl or dialkylamino group, an alkoxy group, a fluoromethyl ketone or a chloromethyl-ketone at the C-terminal end.

5. The medical product according to claim 1, wherein X represents an oligopeptide comprising at least one amino acid having D configuration.

6. The medical product according to claim 1, further comprising at least one anti-inflammatory, anti-prolific, anti-thrombotic, and/or anti-coagulative agent.

7. A method for the preparation of a hemocompatibly coated medical product, comprising the steps of:
a) providing a surface of a medical product,
b) coating said surface with a coating composition comprising at least one compound of formula R-Lys-X as defined by claim 1.

8. A method for the preparation of a hemocompatibly coated medical product, comprising the steps of:
a) providing a surface of a medical product,
b') coating said surface with a first layer comprising biologically stable and/or biodegradable polymers and
b") coating said first layer with a coating composition comprising at least one compound of formula R-Lys-X as defined by claim 1.

9. The method according to claim 7 or 8, wherein the layer of biologically stable and/or biodegradable polymers and/or the layer containing said compound of general formula R-Lys-X further comprises an anti-inflammatory, anti-prolific, anti-thrombotic, and/or anti-coagulative agent.

10. A coated medical product obtained according to the method of any one of claims 7 - 9.

## Patentansprüche

1. Ein medizinisches Produkt beschichtet mit einer pharmazeutischen Zusammensetzung umfassend eine Verbindung mit der Formel R-Lys-X und mindestens einer pharmazeutisch geeigneten Trägersubstanz, Polymer-Matrix, Lösungsmittel und/oder Verdünnungsmittel, wobei X ein Oligopeptid repräsentiert, das ausgewählt wird aus der Gruppe umfassend Pro-Thr, Pro-Ala, Pro-Arg, Pro-Asn, Pro-Asp, Pro-Cys, Pro-Glu, Pro-Gln, Pro-Gly, Pro-His, Pro-Ile, Pro-Leu, Pro-Lys, Pro-Met, Pro-Phe, Pro-Pro, Pro-Ser, Pro-Trp, Pro-Thr-Thr, Pro-Thr-Val, Pro-Thr-Ala, Pro-Thr-Arg, Pro-Thr-Asn, Pro-Thr-Asp, Pro-Thr-Cys, Pro-Thr-Glu, Pro-Thr-Gln, Pro-Thr-Gly, Pro-Thr-His, Pro-Thr-Ile, Pro-Thr-Leu, Pro-Thr-Lys, Pro-Thr-Met, Pro-Thr-Phe, Pro-Thr-Pro, Pro-Thr-Ser und Pro-Thr-Trp und wobei R ein Peptid repräsentiert umfassend das Tetrapeptid His-Phe-Arg-Trp und/oder wobei R ein Peptid repräsentiert umfassend das Tripeptid Phe-Arg-Trp und/oder wobei R ein Peptid repräsentiert umfassend das Tripeptid His-Phe-Arg.

2. Das medizinische Produkt gemäß Anspruch 1, wobei R ein Peptid repräsentiert umfassend mindestens eine Aminosäure mit D-Konfiguration und/oder wobei R ein Peptid repräsentiert bestehend aus einer Aminosäure mit D-Konfiguration und/oder R ein Peptid repräsentiert, welches eine Acylgruppe oder Acetylgruppe am N-terminalen Ende trägt.

3. Das medizinische Produkt gemäß Anspruch 1, wobei X das Oligopeptid Pro-Thr repräsentiert.

4. Das medizinische Produkt gemäß Anspruch 1 oder 3, wobei X ein Oligopeptid repräsentiert, welches eine Aminosäure, eine Monoalkyl- oder Dialkylgruppe, eine Alkoxygruppe, ein Fluormethylketon oder ein Chlormethylketon am C-terminalen Ende trägt.

5. Das medizinische Produkt gemäß Anspruch 1, wobei X ein Oligopeptid repräsentiert umfassend mindestens eine Aminosäure mit D-Konfiguration.

6. Das medizinische Produkt gemäß Anspruch 1, ferner umfassend mindestens einen anti-inflammatorischen, anti-proliferativen, anti-thrombotischen und/oder anti-koagulativen Wirkstoff.

7. Ein Verfahren zur Herstellung eines hämokompatibel beschichteten Medizinprodukts, umfassend die Schritte:
a) Bereitstellung einer Oberfläche eines medizinischen Produkts
b) Beschichtung der besagten Oberfläche mit einer Beschichtungszusammensetzung umfassend mindestens eine Verbindung der Formel R-Lys-X wie in Anspruch 1 definiert.

8. Ein Verfahren zur Herstellung eines hämokompatibel beschichteten Medizinprodukts umfassend die Schritte:
a) Bereitstellung einer Oberfläche eines medizinischen Produkts
b') Beschichtung der besagten Oberfläche mit einer ersten Schicht umfassend biologisch stabile und/oder biodegradierbare Polymere und
b") Beschichtung der besagten ersten Schicht mit einer Beschichtungszusammensetzung umfassend mindestens eine Verbindung der Formel R-Lys-X wie in Anspruch 1 definiert.

9. Ein Verfahren gemäß Anspruch 7 oder 8, wobei die Schicht mit dem biologisch stabilen und/oder biodegradierbaren Polymeren und/oder die Schicht, welches die besagte Verbindung der allgemeinen Formel R-Lys-X enthält, zusätzlich einen anti-inflammatorischen, anti-thrombotischen und/oder anti-koagulativen Wirkstoff umfasst.

10. Ein beschichtetes Medizinprodukt erhalten nach einem der Verfahren gemäß einem der Ansprüche 7 - 9.

## Revendications

1. Un produit médical revêtu avec une composition pharmaceutique comprenant un composé de formule R-Lys-X et au moins un véhicule, une matrice de polymère, un solvant et/ou un diluant pharmaceutiquement acceptable, où X représente un oligopeptide sélectionné à partir du groupe comprenant Pro-Thr, Pro-Ala, Pro-Arg, Pro-Asn, Pro-Asp, Pro-Cys, Pro-Glu, Pro-Gln, Pro-Gly, Pro-His, Pro-Ile, Pro-Leu, Pro-Lys, Pro-Met, Pro-Phe, Pro-Pro, Pro-Ser, Pro-Trp, Pro-Thr-Thr, Pro-Thr-Val, Pro-Thr-Ala, Pro-Thr-Arg, Pro-Thr-Asn, Pro-Thr-Asp, Pro-Thr-Cys, Pro-Thr-Glu, Pro-Thr-Gln, Pro-Thr-Gly, Pro-Thr-His, Pro-Thr-Ile, Pro-Thr-Leu, Pro-Thr-Lys, Pro-Thr-Met, Pro-Thr-Phe, Pro-Thr-Pro, Pro-Thr-Ser, et Pro-Thr-Trp et où R représente un peptide comprenant le tétrapeptide His-Phe-Arg-Trp et/ou R représente un peptide comprenant le tripeptide Phe-Arg-Trp et/ou R représente un peptide comprenant le tripeptide His-Phe-Arg.

2. Le produit médical selon la revendication 1, où R représente un peptide comprenant au moins un acide aminé ayant la configuration D et/ou où R représente un peptide consistant en acides aminés ayant la configuration D et/ou R représente un peptide portant un groupement acyle ou acétyle à l'extrémité N-terminale.

3. Le produit médical selon la revendication 1, où X représente l'oligopeptide Pro-Thr.

4. Le produit médical selon la revendication 1 ou 3, où X représente un oligopeptide portant à l'extrémité C-terminale un groupement amino, un groupement monoalkyl- ou dialkylamino, un groupement alcoxy, une fluorométhyl-cétone ou une chlorométhyl-cétone.

5. Le produit médical selon la revendication 1, où X représente un oligopeptide comprenant au moins un acide aminé ayant la configuration D.

6. Le produit médical selon la revendication 1, comprenant de plus au moins un agent anti-inflammatoire, anti-prolifique, anti-thrombotique, et/ou anti-coagulant.

7. Un procédé pour la préparation d'un produit médical revêtu hémocompatiblement, comprenant les étapes suivantes:
a) fournir une surface d'un produit médical,
b) couvrir ladite surface avec une composition de revêtement comprenant au moins un composé de formule R-Lys-X tel que défini à la revendication 1.

8. Un procédé pour la préparation d'un produit médical revêtu hémocompatiblement, comprenant les étapes suivantes:
a) fournir une surface d'un produit médical,
b') couvrir ladite surface avec une première couche comprenant des polymères biologiquement stables et/ou biodégradables, et
b") couvrir ladite première couche avec une composition de revêtement comprenant au moins un composé de formule R-Lys-X tel que défini à la revendication 1.

9. Le procédé selon la revendication 7 ou 8, où la couche de polymères biologiquement stables et/ou biodégradables et/ou la couche contenant ledit composé de formule R-Lys-X comprend de plus un agent anti-inflammatoire, anti-prolifique, anti-thrombotique, et/ou anti-coagulant.

10. Un produit médical revêtu obtenu selon le procédé de l'une quelconque des revendications 7 - 9.
